# EUROPEAN PATENT SPECIFICATION

(11) **EP 1 136 563 B1**
(45) Date of publication and mention of the grant of the patent: **07.06.2006**
(21) Application number: 01201027.8
(22) Date of filing: 20.03.2001
(51) Int. Cl.: C12Q 1/04, G01N 1/30

(54) **Method of staining, and detecting and counting bacteria**
Verfahren zur Färbung, zum Nachweis und Zählen von Bakterien
Méthode de coloration, détection et énumération des bactéries

(30) Priority: 22.03.2000 JP 2000080998
(43) Date of publication of application: 26.09.2001
(73) Proprietor: SYSMEX CORPORATION, Chuo-ku Kobe 651-0073 (JP)
(72) Inventor: Inoue, Junya, Sysmex Corporation, Kobe-shi, Hyogo 651-0073 (JP); Kawashima, Yasuyuki, Sysmex Corporation, Kobe-shi, Hyogo 651-0073 (JP); Ikeuchi, Yoshiro, Sysmex Corporation, Kobe-shi, Hyogo 651-0073 (JP)
(74) Representative: Goulard, Sophie

(56) References cited:
- EP-A- 0 841 403
- US-A- 2 168 630
- US-A- 4 622 298
- US-A- 4 693 972
- US-A- 5 798 221
- IRKHIN A.I. ET AL.: "Sensitivity of Escherichia Coli cell membranes to various classes of detergents" MIKROBIOLOGIIA, vol. 58, no. 2, 1989, pages 217-221, XP009021879

## Description

### BACKGROUND OF THE INVENTION

### Field of the Invention

The present invention relates to a reagent for staining bacteria in urine samples, and to a method for detecting and counting bacteria in urine samples using such a reagent.

### Related Art

The number of bacteria in urine is an important parameter in clinical diagnosis to judge the presence of infection. In general, the presence of bacteria of 10⁵ or more/ml in urine is recognized as a criterion of positive urinary tract infection. If urine contains bacteria of 10³ or more/ml, it is diagnosed as contaminated urine (normal bacteria flora), i.e., negative urinary tract infection. If bacteria of about 10⁴/ml is observed, the diagnosis is reserved but the sample is often re-examined.

Conventionally, observation of bacteria in urine has been performed by microscopic examination of Gram stained bacteria, unstained bacteria without Gram staining treatment or fluorescence-stained bacteria.

Urine often contains contaminants such as mucus threads, crystals, amorphous salts and cell fragments that are clinically insignificant. These substances hinder the measurement of significant particles (in particular bacteria) so that it has been difficult to accurately count the number of bacteria. Actually, there has been no method of counting bacteria of about 10⁴/ml, accurately.

In the case of Gram stain, bacteria and contaminants are stained simultaneously so that counting loss of bacteria of a small number occurs frequently in the microscopic examination. Further, Gram stain includes a number of staining steps and takes time (about 15 minutes) so that working efficiency is poor.

The microscopic examination of bacteria without staining treatment can be carried out quickly, but it cannot discriminate bacteria particularly when coccus contaminants are contained.

The microscopic examination of fluorescence-stained bacteria shows better detectability than the above-mentioned two methods. However, there has not been established how to eliminate other contaminants than bacteria and to stain the bacteria quickly.

Agar medium method, which is a standard method, requires 16 hours or more to determine the bacteria number, so that it cannot be regarded as a quick method.

USP 4,622,298 and Japanese Unexamined Patent Publication No. Hei 9 (1997)-119926 each proposes a method of detecting bacteria in a fluorescence-stained urine sample with a flow cytometer. However, in the above references, examination of a sample including contaminants is not conducted.

Japanese Unexamined Patent Publication No. Hei 9 (1997)-329596 describes an analysis of solid components in urine which are difficult to be discriminated from each other. In this method, a urine sample is treated with an aqueous solution containing a surfactant and examined with a flow cytometer. However, it does not describe a method of discrimination between bacteria and contaminants.

EP 0841403 discloses a method of staining and measuring the number of bacteria wherein a sample is mixed with a coloring composition comprising a dye and a surfactant, preferably a non-ionic surfactant. This coloring composition must be controlled to have a pH of from 4 to 6 for stability purpose and is also used in this range of pH for staining bacteria.

US 2002/168630 is related to methods and kits for detecting and quantifying viable cells, such as bacteria, in a sample using fluorescent dyes that can be internalized predominantly by viable cells and have properties measurably altered when bound to target compound. The sample to be tested can be a bodily fluid or food products. Among the steps of the method as disclosed, the sample is treated with an agent that affects a cell membrane property such as a detergent.

US 5,798,221 relates to a method for conditioning samples such as milk or meats, containing fat globules and somatic cells and/or protein particles before they are subjected to fluorescence measurements to determine the bacterial content and method for performing the determination of bacterial content in such samples. The method comprises treating the initial sample with an ion-chelating agent, a proteolytic enzyme, a detergent and a bacteriologically specific fluorochrome. The pH of the sample being treated is preferably kept in the range of 6-11 by the means of a buffer.

### SUMMARY OF THE INVENTION

An object of the present invention is to provide a reagent for staining bacteria in urine samples which allows quick and efficient detection of bacteria without performing cultivation even if a sample contains contaminants.

The present invention provides a reagent for staining bacteria in an urine sample comprising an aqueous solution to be mixed with the sample, containing a cationic surfactant to accelerate dye transmissivity of bacteria and an inorganic salt selected from the group consisting of sulfate and nitrate, having a pH of 2.0 to 4.5; and a staining solution containing a dye for staining the bacteria.

Further, the present invention provides a method of detecting and counting bacteria comprising the following steps of:
(1) mixing an urine sample with the reagent as described in the above;
(2) introducing the thus treated sample into a detecting part of a flow cytometer and irradiating cells of the stained bacteria one by one with light to measure scattered light and fluorescent light emitted from each of the cells; and
(3) discriminating the bacteria from other components in accordance with an intensity of a scattered light signal and an intensity of a fluorescent light signal or a pulse width reflecting the length of particles to count the number of the bacteria.

### BRIEF DESCRIPTION OF THE DRAWINGS

Fig. 1 is a scattergram of a fluorescent light intensity - a forward scattered light intensity obtained in the case where cultivated E. coli was examined in Example 1 of the present invention;
Fig. 2 is a scattergram of a fluorescent light intensity - a forward scattered light intensity obtained in the case where various cultivated bacteria are examined in Example 2 of the present invention;
Fig. 3 is a graph illustrating the results of a dilution linearity test performed in Example 3 of the present invention;
Fig. 4 is a graph illustrating correlation between measurement results according to the present invention and those of a CLED medium cultivation obtained in Example 4 of the present invention; and

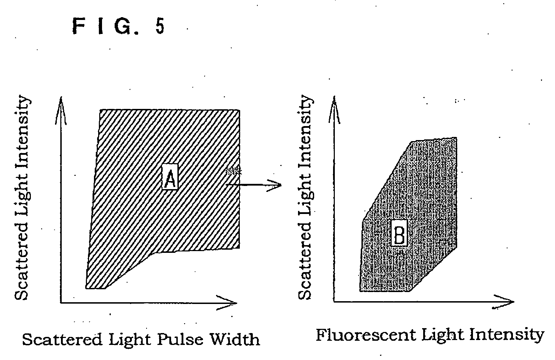

Fig. 5 is a view illustrating the outline of the method of detecting bacteria according to the present invention.

### DESCRIPTION OF THE PREFERRED EMBODIMENTS

In the present invention, the urine sample is a sample to be examined for the presence or absence of bacteria and to count a number of bacteria if the sample contains bacteria. Bacteria referred herein include bacteria observed in a urine sample such as E.coli, Klebsiella sp., as well as Staphylococcus sp., Pseudomonas sp., Serratia sp., Enterobacter sp., Enterococcus sp., Streptococcus sp. and Citrobacter sp. The sample may be diluted with purified water or the like two or more times, preferably 4 to 15 times, more preferabley 5 to 10 times.

No particular limitation is given to the cationic surfactant, but preferably used is a quarternary ammonium salt represented by the following formula: 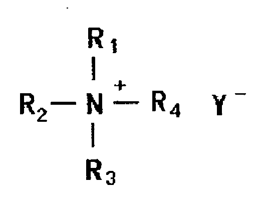 wherein R₁ is a C₈₋₁₈ alkyl group; R₂, R₃ and R₄, the same or different, are a C₁₋₃ alkyl group or benzyl group; Y⁻ is a halogen ion.

The C₁₋₃ alkyl group may be methyl, ethyl, propyl and the like. The C₈₋₁₈ alkyl group may be octyl, decyl, dodecyl, tetradecyl and the like.

For example, suitably used are decyl trimethyl ammonium salt, dodecyl trimethyl ammonium salt, tetradecyl trimethyl ammonium salt, hexadecyl trimethyl ammonium salt, octadecyl trimethyl ammonium salt and the like. Appropriate use amount thereof may be 10 to 30000 mg/l, preferably 100 to 3000 mg/l in the sample (final concentration).

Addition of the cationic surfactant to the bacteria-containing sample damages cell membrane of bacteria so that a dye easily enters the cell. As a result, content of the bacteria cell and the dye are efficiently bonded to each other so that bacteria are stained well, which facilitates the discrimination of bacteria from contaminants. Further, mucus threads, erythrocytes and cell fragments are lysed or shrunk, which reduces influence thereof on the bacteria detection.

The dye is not particularly limited as long as it can stain bacteria. A dye capable of staining bacteria under an acidic state is preferably used. The concentration thereof may suitably be determined depending on the kind of dye, for example, in the range of 0.1 to 100 ppm (final concentration). In view of bacteria detectability, a fluorescent dye which is at least bonded to one of components constituting bacteria and emits fluorescent light is advantageously used. From this point of view, polymethine dyes are preferable. For example, the following dyes (1) to (11) are used:
(1) Thiazole Orange;
(2); 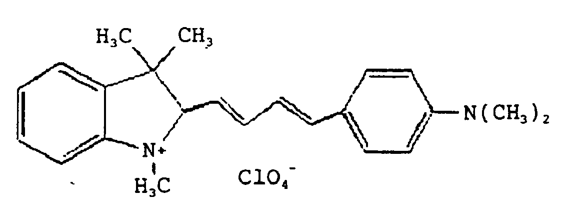
(3); 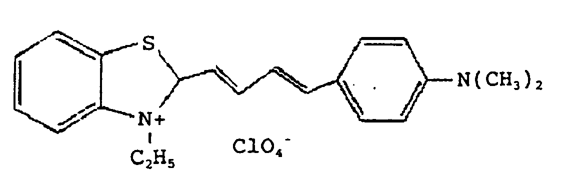
(4); 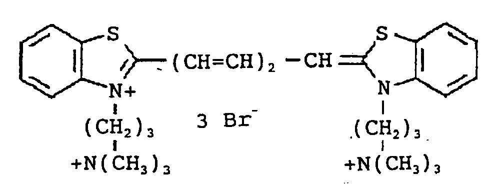
(5); 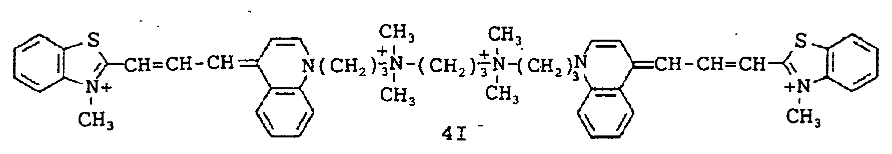
(6); 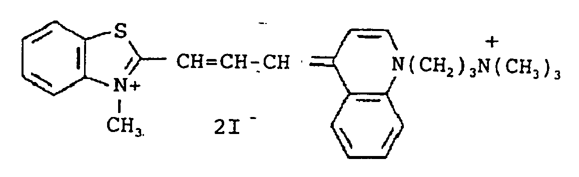
(7); 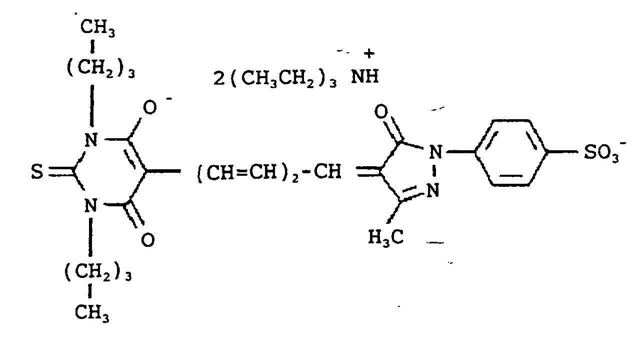
(8); 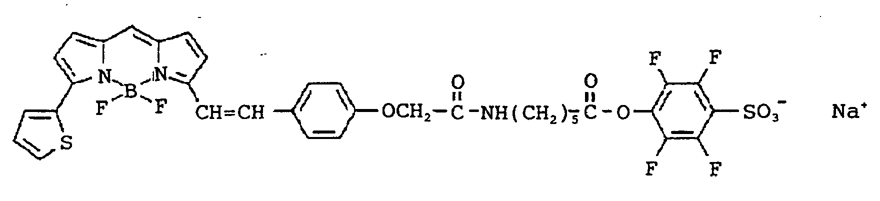
(9); 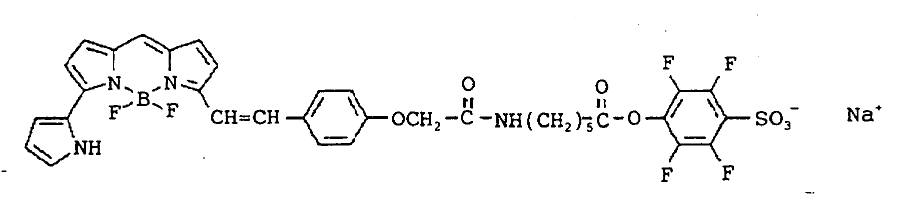
(10) a compound represented by the following general formula: 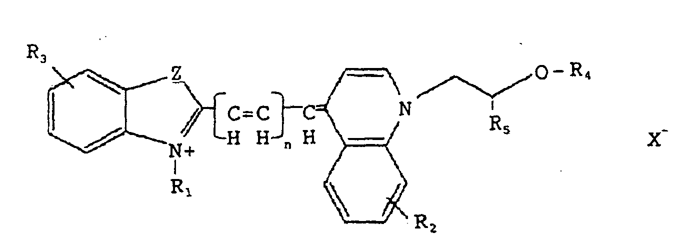 wherein R₁ is a hydrogen atom or a C₁₋₃ alkyl group; R₂ and R₃ are a hydrogen atom, a C₁₋₃ alkyl group or a C₁₋₃ alkoxy group; R₄ is a hydrogen atom, an acyl group or a C₁₋₃ alkyl group; R₅ is a hydrogen atom or a C₁₋₃ alkyl group which may be substituted; Z is a sulfur atom, an oxygen atom or a carbon atom substituted with a C₁₋₃ alkyl group; n is 1 or 2; X⁻ is an anion; and
(11) a compound represented by the following general formula: 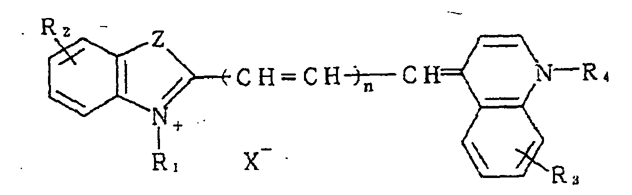 wherein R₁ is a hydrogen atom or a C₁₋₁₈ alkyl group; R₂ and R₃ are a hydrogen atom, a C₁₋₃ alkyl group or a C₁₋₃ alkoxy group; R₄ is a hydrogen atom, an acyl group or a C₁₋₁₈ alkyl group; Z is sulfur, oxygen or a carbon atom substituted with a C₁₋₃ alkyl group; n is 0, 1 or 2; X⁻ is an anion.

The C₁₋₃ alkyl group may be methyl, ethyl, propyl and the like. The C₁₋₁₈ alkyl group may be methyl, ethyl, propyl, octyl, decyl, dodecyl, tetradecyl and the like. The a C₁₋₃ alkoxy group may be methoxy, ethoxy, propoxy and the like. Substituents to the C₁₋₃ alkyl group may be a hydroxyl group, a halogen atom and the like.

Among the above-mentioned dyes, (1) is commercially available. (2) and (3) are supplied by Nippon Photosensitive Dye Laboratory Ltd., and (5) to (9) are supplied by Molecular Probes, Inc. Manufacturing methods of (10) and (11) are described in Japanese Unexamined Patent Publications Nos. Hei 9(1997)-104683 and Hei 10(1998)-319010, respectively.

Among the dyes (10), a dye represented by the formula: 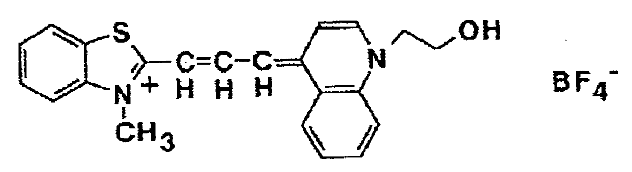 is particularly suitable.

Further, among the dyes (11), a dye represented by the formula: 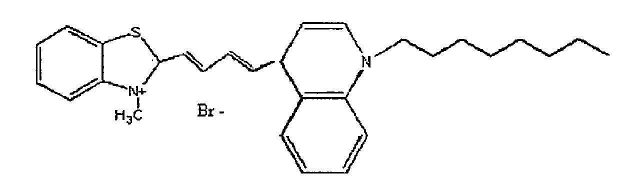 is particularly suitable.

In the present invention, pH at the staining step is not specifically limited as long as it allows the bacteria staining. The urine sample is stained at an acidic pH, preferably pH 2 to 4.5, more preferably pH 2 to 3, (1) bacteria is stained better than in a neutral or alkaline state and (2) nonspecific staining of mucus threads is prevented and the mucus threads is lysed to a certain extent. Thus, the acidic state is advantageous to the bacteria staining.

A buffer of pKa 1 to 5.5 is used to maintain the acidic state. The buffer is not particularly limited, but an acid or a compound capable of maintaining pH 2.0-3.0 may be used. As the buffer, it may be utilized one or more kinds of compounds selected from the group comprising of: citric acid or its salts, phosphoric acid or its salts, phthalic acid or its salts, succinic acid or its salts, lactic acid or its salts, ε-aminocaproic acid or its salts, fumaric acid or its salts, β-alanine, glycine and the like. The salts described above include alkali or alkaline earth salts. Suitable examples thereof is at least one selected from the group consisting of: citric acid-NaOH, citric acid -sodium citrate, potassium dihydrogen phosphate-disodium hydrogen phosphate, potassium dihydrogen phosphate-NaOH, citric acid-disodium hydrogen phosphate, potassium hydrogen phthalate-NaOH, succinic acid-NaOH, lactic acid-NaOH, ε-aminocaproic acid-HCl, fumaric acid-HCl, β-alanine-NaOH, glycine-NaOH and the like. Appropriate use amount thereof is such that the above-mentioned pH range is maintained, preferably about 10 to 500 mM in the sample.

The staining is carried out by further utilizing an inorganic salt of either sulfate or nitrate, thus the fluorescent dye transmissivity of bacteria is enhanced and nonspecific staining of contaminants is prevented. The inorganic salt may be used in a concentration of about 10 to 500 mM, preferably about 50 to 200 mM in the sample.

The method of detecting and counting bacteria according to the present invention is carried out by the steps of:
(1) mixing an urine sample with the reagent as described above,
(2) introducing the thus treated sample into a detecting part of a flow cytometer and irradiating cells of the stained bacteria one by one with light to measure scattered light and fluorescent light emitted from each of the cells; and
(3) discriminating the bacteria from other components in accordance with an intensity of a scattered light signal and an intensity of a fluorescent light signal or a pulse width reflecting the length of particles to count the bacteria.

In the present invention, the working a dye on the urine sample (staining) may be carried out by mixing the sample, one by one or simultaneously, an aqueous solution containing the cationic surfactant and the inorganic salt and a solution containing the dye. The dye may be contained in the aqueous solution containing the cationic surfactant and the inorganic salt. However, where the dye to be utilized is unstable in the aqueous solution, it may be dissolved in a water-soluble organic solvent such as methanol, ethanol or ethylene glycol and then mixed for use with the aqueous solution containing the substance capable of reducing nitrite ions and/or the cationic surfactant. This improves storage stability of the dye.

Temperature and time for the staining are not particularly limited, but the staining may be performed at about 15 to 50°C for about 20 minutes or less, preferably about 15 minutes or less, more preferably about 15 minutes immediately after the mixing.

The sample stained by the method of the present invention may be observed with a microscope or an imaging apparatus to detect bacteria. Alternatively, bacteria can be detected and counted by using a flow cytometer with high accuracy. The flow cytometer used herein may be a commercially available apparatus generally utilized in the art.

Discrimination of bacteria from other components and counting of bacteria are carried out in accordance with combination of signals obtained by using a flow cytometer. Example of the combination includes, for example, a forward scattered light intensity and a forward scattered light pulse width, a forward scattered light intensity and a fluorescent light intensity, a forward scattered light pulse width and a fluorescent light intensity, and the like. In a suitable manner, for example, firstly, a scattergram is formed from the combination of the forward scattered light intensity and the forward scattered light pulse width, and then gating is performed to a mass including bacteria specified on the scattergram to separate mucus threads, mainly. Further, another scattergram is formed from the forward scattered light intensity and the fluorescent light intensity of the gated mass to separate bacteria from other components (crystals, cell fragments and the like) based on the difference in the fluorescent light intensity. The outline of the method is shown in Fig. 7. Where the sample contains bacteria only, a scattergram is formed from the forward scattered light intensity and the fluorescent light intensity to count them.

### Examples

Hereinafter, preferred examples of the method of staining and detecting bacteria according to the present invention are described, but the present invention is not limited thereto.

### Example 1

### Reagent Composition

### (Diluent)

| | |
|---|---|
| Citric acid | 100 mM |
| NaOH | up to pH 4.1 |
| Tetradecyl trimethyl ammonium bromide | 0.1 %(w/v) |

### (Staining solution)

| | |
|---|---|
| Dye A (of the above-mentioned formula). | 40 ppm (in ethylene glycol) |

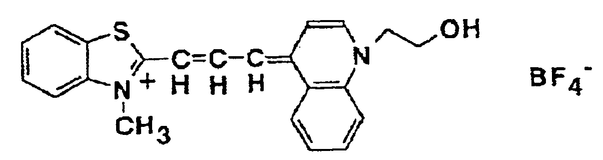

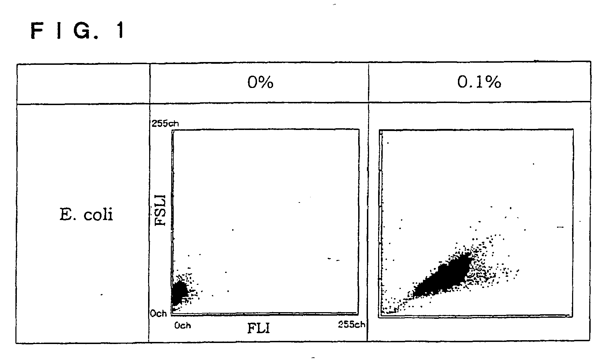

To 100 µl of a sample containing cultivated E. coli, 1000 µl of the above-mentioned diluent was added and the staining solution was added so that the final concentration of the dye A would be 1 ppm. Staining was carried out at 40°C for 30 seconds and then scattered light and fluorescent light were measured by the flow cytometer provided with a red semiconductor laser as a light source (amount of examined urine: 7.8 µl). As a control, measurement was performed using a reagent in which tetradecyl trimethyl ammonium bromide was not contained. The results are shown in Fig. 1.

In the case where the reagent without tetradecyl trimethyl ammonium bromide was used (left scattergram), the fluorescent light intensity was 30 ch or less, i.e., few E. coli was stained. In contrast, where the reagent contained 0.1% (w/v) tetradecyl trimethyl ammonium bromide (right scattergram), a mass of E. coli was distributed around the fluorescent light intensity of 50 ch or more.
Thus, it was observed that dye transmissivity was enhanced.

### Example 2

### Reagent Composition

### (Diluent)

| | |
|---|---|
| Citric acid | 100 mM (pH 2.5) |
| Tetradecyl trimethyl ammonium bromide | 0.1 %(w/v) |
| Sodium sulfate | 90 mM |

### (Staining solution)

### Same as Example 1

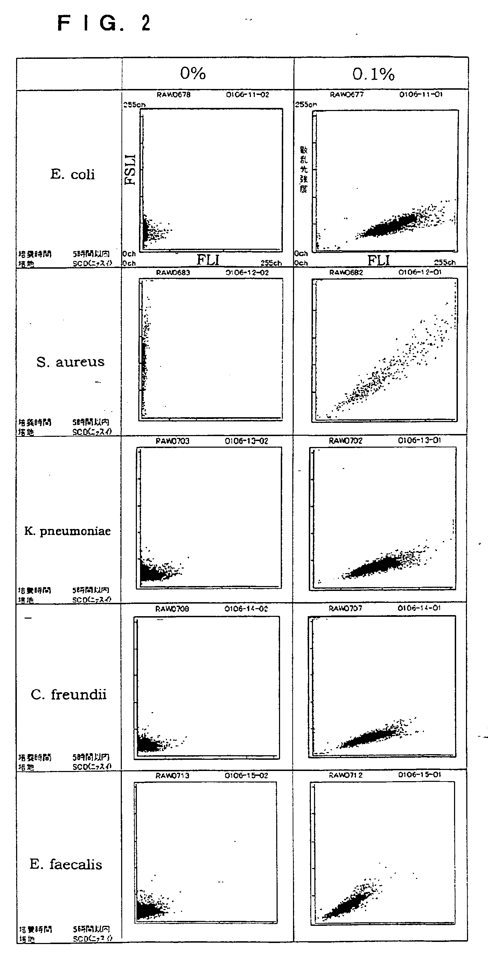

With the above-mentioned reagent, samples each containing cultivated bacteria (E.coli, S.aureus, K.pneumoniae, C.freundii, E.faecalis) were examined in the same manner as in Example 1. The results are shown in Fig. 2.

Where the reagent without tetradecyl trimethyl ammonium bromide was used, the fluorescent light intensity in the samples was weak, i.e., few bacteria were stained. However, the addition of tetradecyl trimethyl ammonium bromide caused increase in the fluorescent light intensity and thus it was observed the bacteria were stained well. In comparison of E. coli measurement between the present Example in which sodium sulfate was further added and Example 1 in which sodium sulfate was not contained, it is shown that the fluorescent light intensity was more enhanced in the present Example, i.e., the addition of sodium sulfate was effective.

### Example 3

### Dilution linearity

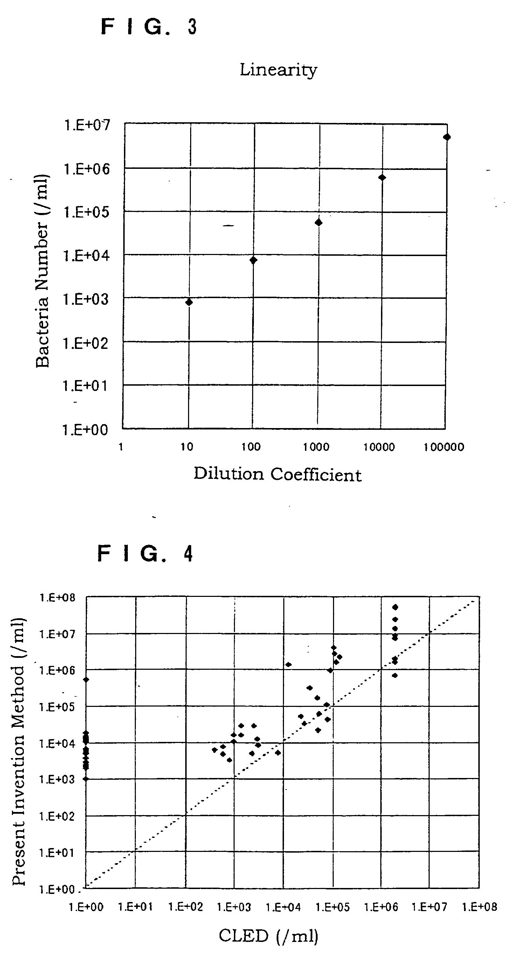

E. coli was cultivated and diluted samples each diluted at dilution coefficient of 1, 10, 100, 1000, 10000 and 100000 were prepared to perform measurement by utilizing the reagent used in Example 2. Fig. 3 shows the results. The bacteria number was obtained by gating a mass including bacteria specified on the scattergram formed from the forward scattered light intensity and the fluorescent light intensity

As shown in Fig. 3, favorable linearity was obtained in the bacteria concentration range of 10³ to 10⁷/ml.

### Example 4

### Examination of urine sample

With the reagent used in Example 2, 62 urine samples were examined and consideration was given to the correlation between the results thereof and those obtained by cultivation on a CLED medium performed as a control.

For measurement of the bacteria number, a scattergram was formed from a combination of the forward scattered light intensity and the forward scattered light pulse width and then gating was performed to a mass including bacteria specified on the scattergram. Then, another scattergram was formed from a combination of the forward scattered light intensity and the fluorescent light intensity of the gated mass. A region of bacteria was specified from the difference in the fluorescent light intensity to count bacteria in the specified region. The results are shown in Fig. 4.

Favorable correlation with the results of the cultivation on the CLED medium. In Fig. 4, several points are observed along the vertical axis. This is because bacteria which hardly grow on medium (static bacteria; bacteria whose growth is hindered by agents or the like) and dead bacteria are also measured in the present invention, though the cultivation on the CLED medium detects living bacteria only.

According to the method of staining bacteria of the present invention, since bacteria are stained in an aqueous state, dry fixation such as Gram staining is not necessarily required. Therefore, staining period can be remarkably reduced and thus a sample for measurement can be prepared in a short time including the staining step.

Since the staining according to the present invention can easily be performed by merely mixing the sample and the reagent, skill required in Gram staining is eliminated. Further, the staining step can be easily carried out, which facilitates the automation through the staining step to the measurement step (such as flow cytometory and image analysis).

According to the method of detecting bacteria of the present invention, bacteria can be counted with high accuracy without being affected by the contaminants. Specifically, bacteria of 10⁴/ml can be counted.

Further, bacteria whose growth is difficult on medium (bacteriostatic samples) can also be counted reliably.

## Claims

1. A reagent for staining bacteria in an urine sample comprising:
an aqueous solution to be mixed with the sample, containing a cationic surfactant and an inorganic salt selected from the group consisting of sulfate and nitrate, having pH of 2.0 to 4.5; and
a staining solution containing a dye for staining the bacteria

2. A reagent according to claim 1, wherein the inorganic salt is used in a concentration of 10 to 500 mM.

3. A reagent according to claim 1, wherein the dye is a fluorescent dye which is at least bonded to one of components constituting the bacteria.

4. A reagent according to claim 1, wherein the dye is at least one selected from the following group consisting of:
(1) Thiazole Orange;
(2) 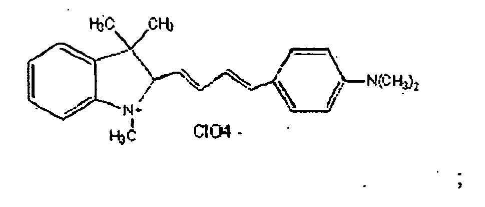
(3) 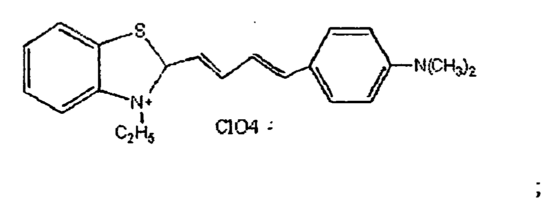
(4) 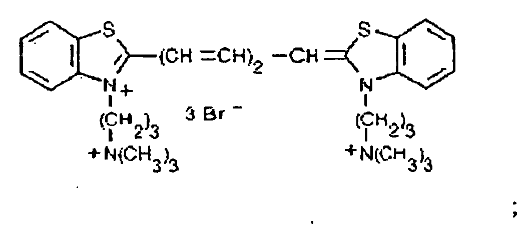
(5) 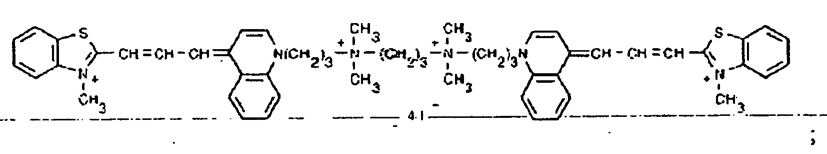
(6) 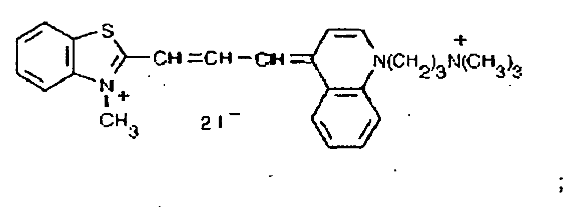
(7) 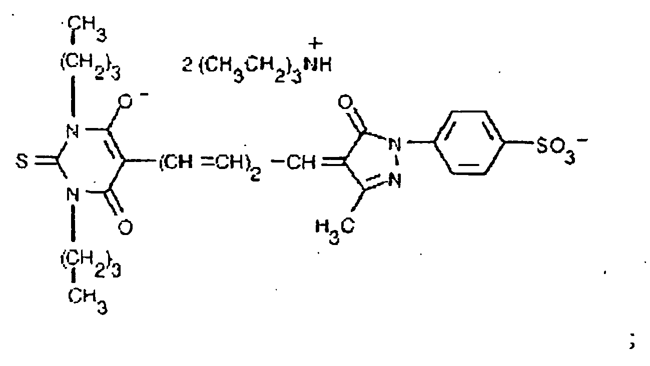
(8) 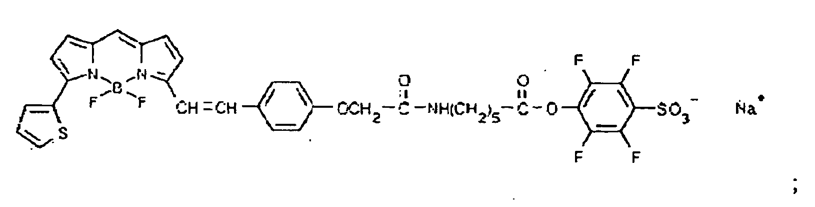
(9) 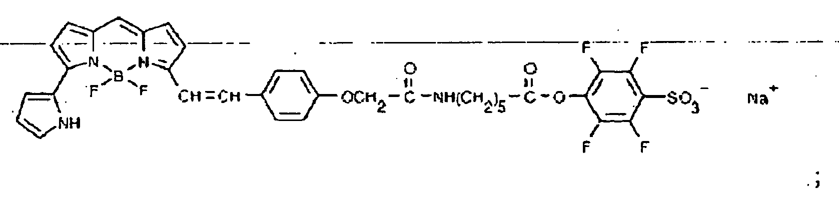
(10) a compound represented by the following general formula: 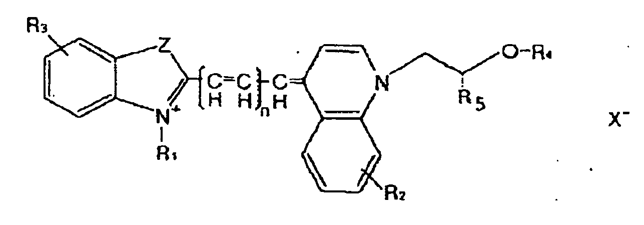 wherein R₁ is a hydrogen atom or a C₁₋₃ alkyl group; R₂ and R₃ are a hydrogen atom, a C₁₋₃ alkyl group or a C₁₋₃ alkoxy group; R₄ is a hydrogen atom, an acyl group or a C₁₋₃ alkyl group; R₅ is a hydrogen atom or a C₁₋₃ alkyl group which may be substituted; Z is a sulfur atom, an oxygen atom or a carbon atom substituted with a C₁₋₃ alkyl group; n is 1 or 2; X⁻ is an anion; and
(11) a compound represented by the following general formula: 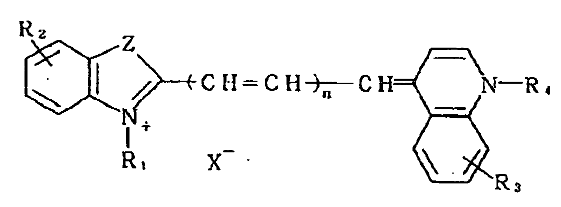
wherein R₁ is a hydrogen atom or a C₁₋₁₈ alkyl group; R₂ and R₃ are a hydrogen atom, a C₁₋₃ alkyl group or a C₁₋₃ alkoxy group; R₄ is a hydrogen atom, an acyl group or a C₁₋₁₈ alkyl group; Z is sulfur, oxygen or carbon having a C₁₋₃ alkyl group; n is 0, 1 or 2; X⁻ is an anion.

5. A reagent according to claim 1, wherein the cationic surfactant is a quaternary ammonium salt represented by the following formula: 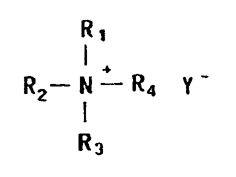 wherein R₁ is a C₈₋₁₈ alkyl group; R₂, R₃ and R₄, the same or different, are a C₁₋₃ alkyl group or a C₁₋₃ benzyl group; Y⁻ is a halogen ion.

6. A reagent according to claim 5, wherein the quaternary ammonium salt is at least one selected from the group consisting of:
decyl trimethyl ammonium salt, dodecyl trimethyl ammonium salt, tetradecyl trimethyl ammonium salt, hexadecyl trimethyl ammonium salt and octadecyl trimethyl ammonium salt.

7. A reagent according to claim 1, wherein the aqueous solution comprises a buffer of pKa 1 to 5.5.

8. A reagent according to claim 7, wherein the buffer is at least one selected from the group consisting of: citric acid-NaOH, citric acid -sodium citrate, potassium dihydrogen phosphate-disodium hydrogen phosphate, potassium dihydrogen phosphate-NaOH, citric acid-disodium hydrogen phosphater, potassium hydrogen phthalate-NaOH, succinic acid-NaOH, lactic acid-NaOH, ε-aminocaproic acid-HCl, fumaric acid-HCl, β-alanine-NaOH and glycine-NaOH.

9. A reagent according to claim 1, wherein the final concentration of the dye is 0.1 to 100 ppm.

10. A reagent according to claim 1, wherein the final concentration of the cationic surfactant is 10 to 30000 mg/l.

11. A reagent according to claim 7, wherein the buffer exists at 10 to 500 mM in the sample.

12. A method of detecting and counting bacteria comprising the following steps of:
(1) mixing an urine sample with the reagent as described in any one of claims 1 to 11;
(2) introducing the thus treated sample into a detecting part of a flow cytometer and irradiating cells of the stained bacteria one by one with light to measure scattered light and fluorescent light emitted from each of the cells; and
(3) discriminating the bacteria from other components in accordance with an intensity of a scattered light signal and an intensity of a fluorescent light signal or a pulse width reflecting the length of particles to count the number of the bacteria.

13. A method according to claim 12, wherein the step (3) of discriminating and counting the bacteria is carried out in accordance with at least one selected from the following combinations of:
(i) a forward scattered light intensity and a forward scattered light pulse width;
(ii) a forward scattered light intensity and a fluorescent light intensity; and
(iii) a forward scattered light pulse width and a fluorescent light intensity.

## Patentansprüche

1. Reagens zum Anfärben von Bakterien in einer Urinprobe, umfassend:
eine wäßrige Lösung, die mit der Probe vermischt werden soll, enthaltend ein kationisches Tensid und ein anorganisches Salz ausgewählt aus der Gruppe bestehend aus Sulfat und Nitrat mit einem pH von 2,0 bis 4,5 und
eine Färbelösung, enthaltend einen Farbstoff zur Anfärbung der Bakterien.

2. Reagens gemäß Anspruch 1, wobei das anorganische Salz in einer Konzentration von 10 bis 500 mM verwendet wird.

3. Reagens gemäß Anspruch 1, wobei der Farbstoff ein fluoreszierender Farbstoff ist, der mindestens an eine der Komponenten, die die Bakterien bilden, gebunden ist.

4. Reagens gemäß Anspruch 1, wobei der Farbstoff mindestens einer ist ausgewählt aus der folgenden Gruppe bestehend aus:
(1) Thiazol-Orange;
(2) 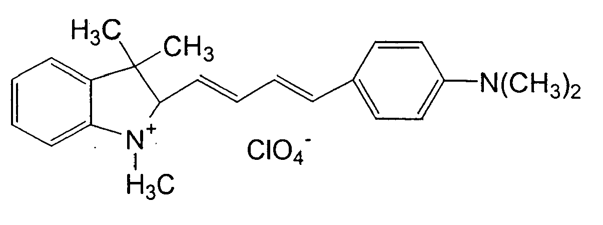
(3) 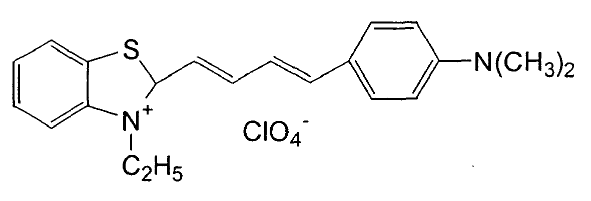
(4) 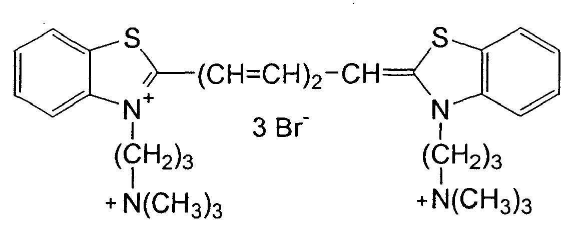
(5) 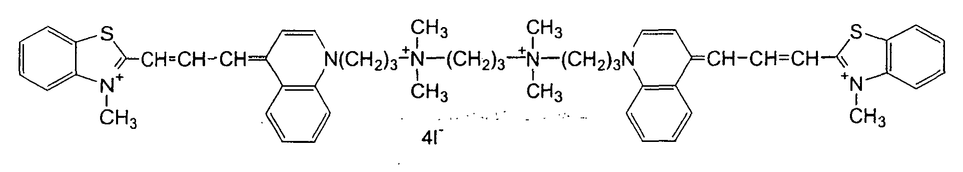
(6) 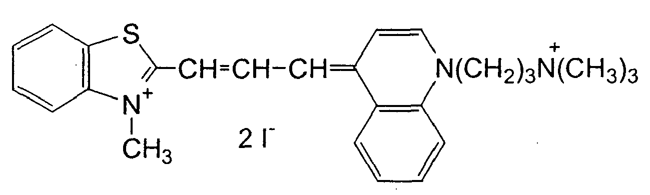
(7) 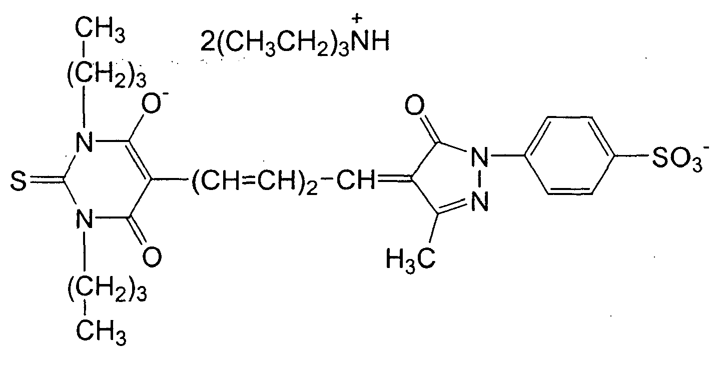
(8) 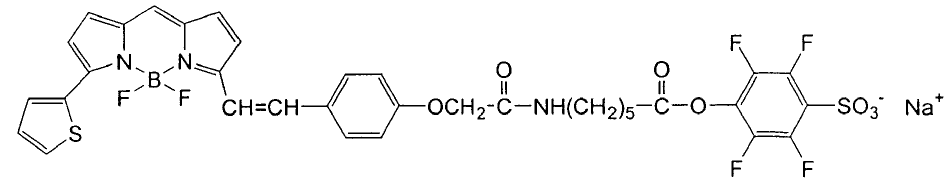
(9) 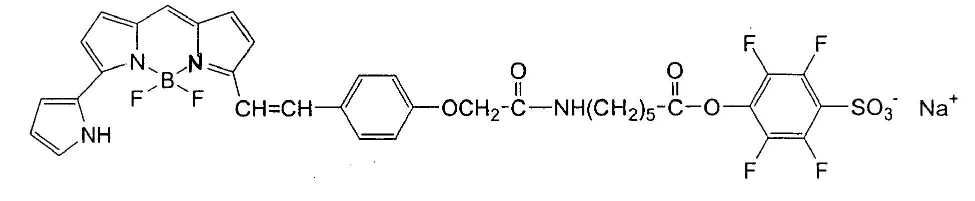
(10) einer Verbindung, dargestellt durch die folgende allgemeine Formel: 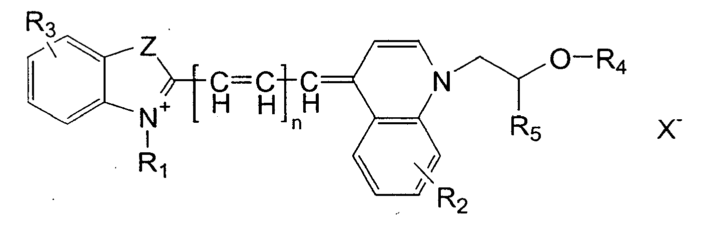 worin R₁ ein Wasserstoffatom oder eine C₁₋₃₋Alkylgruppe ist; R₂ und R₃ sind ein Wasserstoffatom, eine C₁₋₃-Alkylgruppe oder eine C₁₋₃-Alkoxygruppe; R₄ ist ein Wasserstoffatom, eine Acylgruppe oder eine C₁₋₃-Alkylgruppe; R₅ ist ein Wasserstoffatom oder eine C₁₋₃-Alkylgruppe, die substituiert sein kann; Z ist ein Schwefelatom, ein Sauerstoffatom oder ein Kohlenstoffatom substituiert mit einer C₁₋₃-Alkylgruppe; n ist 1 oder 2; X- ist ein Anion; und
(11) einer Verbindung, dargestellt durch die folgende allgemeine Formel: 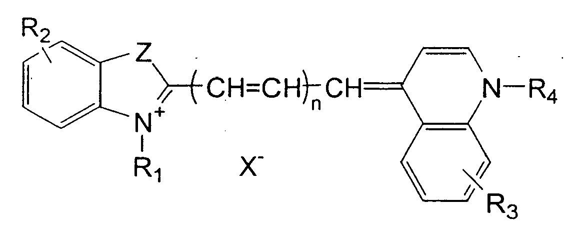
worin R₁ ein Wasserstoffatom oder eine C₁₋₁₈₋Alkylgruppe ist; R₂ und R₃ sind ein Wasserstoffatom, eine C₁₋₃-Alkylgruppe oder eine C₁₋₃-Alkoxygruppe; R₄ ist ein Wasserstoffatom, eine Acylgruppe oder eine C₁₋₁₈-Alkylgruppe; Z ist Schwefel, Sauerstoff oder Kohlenstoff mit einer C₁₋₃-Alkylgrüppe; n ist 0, 1 oder 2; X- ist ein Anion.

5. Reagens gemäß Anspruch 1, wobei das kationische Tensid ein quaternäres Ammoniumsalz, repräsentiert durch die folgende Formel ist: 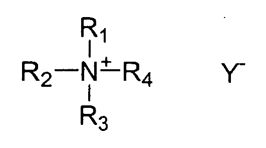 worin R₁ eine C₈₋₁₈-Alkylgruppe ist; R₂, R₃ und R₄ sind dieselben oder unterschiedlich und zwar eine C₁₋₃₋Alkylgruppe oder eine C₁₋₃-Benzylgruppe; Y⁻ ist ein Halogenion.

6. Reagens gemäß Anspruch 5, wobei das quaternäre Ammoniumsalz mindestens eines ist ausgewählt aus der Gruppe bestehend aus Decyltrimethylammoniumsalz, Dodecyltrimethylammoniumsalz, Tetradecyltrimethylammoniumsalz, Hexadecyltrimethylammoniumsalz und Octadecyltrimethylammoniumsalz.

7. Reagens gemäß Anspruch 1, wobei die wäßrige Lösung einen Puffer mit einem pKa von 1 bis 5,5 umfaßt.

8. Reagens gemäß Anspruch 7, wobei der Puffer mindestens einer ist ausgewählt aus der Gruppe bestehend aus Zitronensäure-NaOH, Zitronensäure-natriumcitrat, Kaliumhydrogenphosphat-dinatriumhydrogenphosphat, Kaliumdihydrogenphosphat-NaOH, Zitronensäuredinatriumhydrogenphosphat, Kaliumhydrogenphthalat-NaOH, Bernsteinsäure-NaOH, Milchsäure-NaOH, ε-Aminocapronsäure-HCl, Fumarsäure-HCl, β-Alanin-NaOH und Glycin-NaOH.

9. Reagens gemäß Anspruch 1, wobei die Endkonzentration des Farbstoffs 0,1 bis 100 ppm beträgt.

10. Reagens gemäß Anspruch 1, wobei die Endkonzentration des kationischen Tensids 10 bis 30.000 mg/l beträgt.

11. Reagens gemäß Anspruch 7, wobei der Puffer bei 10 bis 500 mM in der Probe existiert.

12. Verfahren zum Nachweis und Zählen von Bakterien, umfassend die folgenden Schritte:
(1) Vermischen einer Urinprobe mit dem Reagens wie beschrieben in einem der Ansprüche 1 bis 11;
(2) Einführen der so behandelten Probe in einen Nachweisteil eines Flußzytometers und Bestrahlung der Zellen der gefärbten Bakterien eine nach der anderen mit Licht, um gestreutes Licht zu messen und fluoreszierendes Licht, das von jeder der Zellen emittiert wird; und
(3) Unterscheiden der Bakterien von anderen Komponenten gemäß einer Intensität des gestreuten Lichtsignals und einer Intensität eines fluoreszierenden Lichtsignals oder einer Pulsbreite, die die Länge von Teilchen reflektiert, um die Zahl der Bakterien zu zählen.

13. Verfahren gemäß Anspruch 12, wobei der Schritt (3) der Unterscheidung und des Zählens der Bakterien gemäß mindestens einer der folgenden Kombinationen durchgeführt wird:
(i) eine vorwärts gestreute Lichtintensität und eine vorwärts gestreute Lichtpulsbreite;
(ii) eine vorwärts gestreute Lichtintensität und eine fluoreszierende Lichtintensität; und
(iii) eine vorwärts gestreute Lichtpulsbreite und eine fluoreszierende Lichtintensität.

## Revendications

1. Réactif destiné à colorer des bactéries dans un échantillon d'urine comprenant :
une solution aqueuse devant être mélangée avec l'échantillon, contenant un tensioactif cationique et un sel inorganique choisi dans le groupe constitué de sulfate et nitrate, ayant un pH de 2,0 à 4,5 ; et
une solution de coloration contenant un colorant destiné à colorer les bactéries.

2. Réactif selon la revendication 1, dans lequel le sel inorganique est utilisé à une concentration de 10 à 500 mM.

3. Réactif selon la revendication 1, dans lequel le colorant est un colorant fluorescent qui est au moins lié à l'un des composants constituant les bactéries.

4. Réactif selon la revendication 1, dans lequel le colorant est au moins un colorant choisi dans le groupe suivant constitué de :
(1) thiazole orange ;
(2) 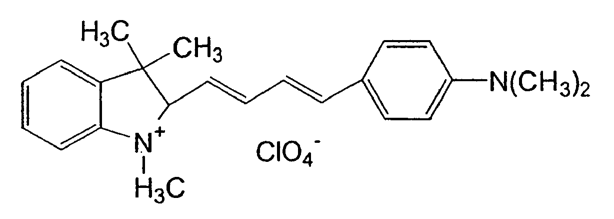
(3) 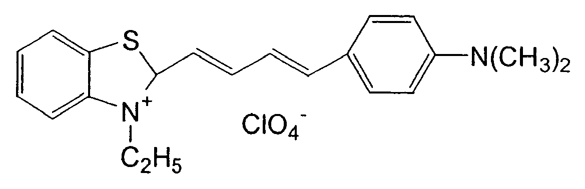
(4) 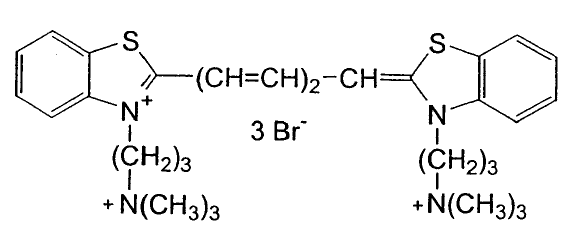
(5) 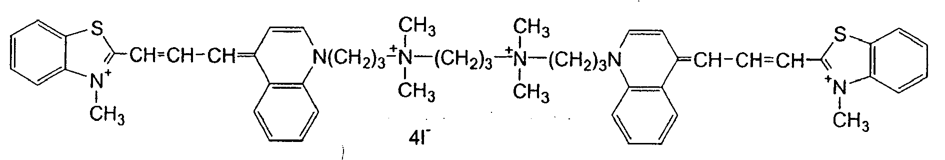
(6) 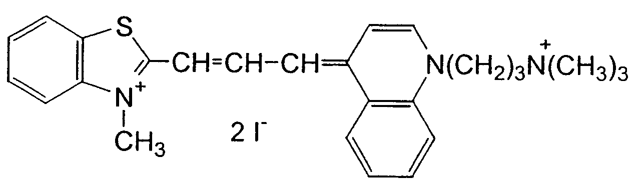
(7) 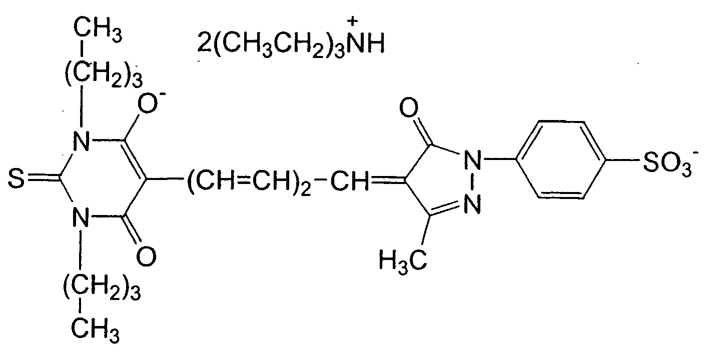
(8) 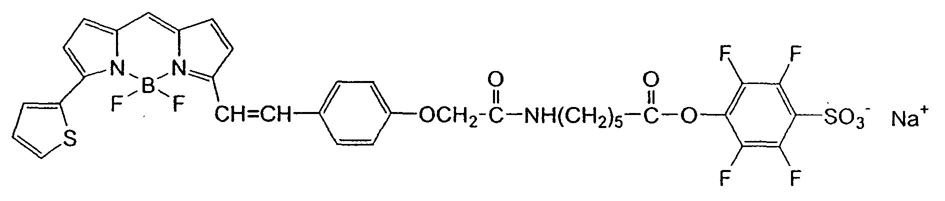
(9) 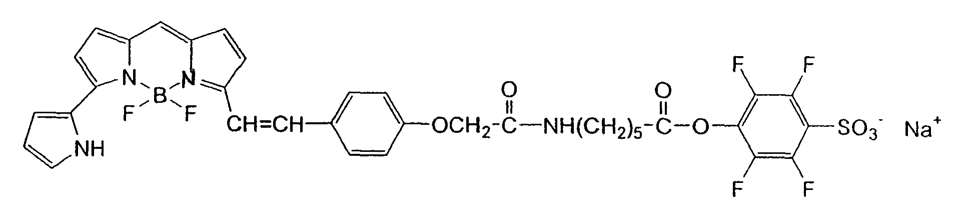
(10) un composé représenté par la formule générale suivante : 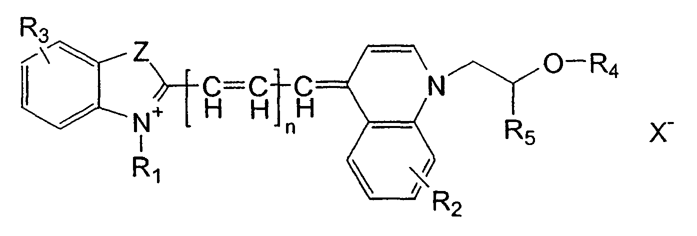 dans laquelle R₁ représente un atome d'hydrogène ou un groupe alkyle en C₁ à C₃ ; R₂ et R₃ représentent un atome d'hydrogène, un groupe alkyle en C₁ à C₃ ou un groupe alcoxy en C₁ à C₃ ; R₄ représente un atome d'hydrogène, un groupe acyle ou un groupe alkyle en C₁ à C₃ ; R₅ représente un atome d'hydrogène ou un groupe alkyle en C₁ à C₃ qui peut être substitué ; Z représente un atome de soufre, un atome d'oxygène ou un atome de carbone substitué avec un groupe alkyle en C₁ à C₃ ; n vaut 1 ou 2 ; X⁻ est un anion ; et
(11) un composé représenté par la formule générale suivante : 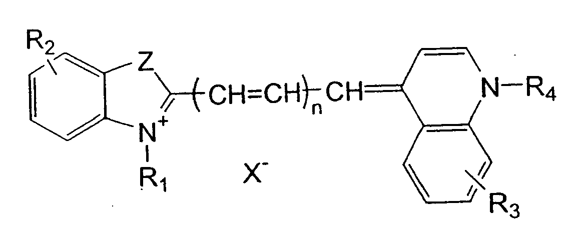
dans laquelle R₁ représente un atome d'hydrogène ou un groupe alkyle en C₁ à C₁₈; R₂ et R₃ représentent un atome d'hydrogène, un groupe alkyle en C₁ à C₃ ou un groupe alcoxy en C₁ à C₃; R₄ représente un atome d'hydrogène, un groupe acyle ou un groupe alkyle en C₁ à C₁₈; Z représente le soufre, l'oxygène ou le carbone comportant un groupe alkyle en C₁ à C₃; n vaut 0, 1 ou 2 ; X⁻ est un anion.

5. Réactif selon la revendication 1, dans lequel le tensioactif cationique est un sel d'ammonium quaternaire représenté par la formule suivante : 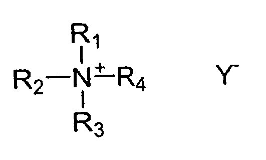 dans laquelle R₁ représente un groupe alkyle en C₈ à C₁₈ ; R₂, R₃ et R₄, identiques ou différents, représentent un groupe alkyle en C₁ à C₃ ou un groupe benzyle en C₁ à C₃ ; Y⁻ est un ion halogène.

6. Réactif selon la revendication 5, dans lequel le sel d'ammonium quaternaire est au moins un sel choisi dans le groupe constitué de : sel de triméthylammonium décylique, sel de triméthylammonium dodécylique, sel de triméthylammonium tétradécylique, sel de triméthylammonium hexadécylique et sel de triméthylammonium octadécylique.

7. Réactif selon la revendication 1, dans lequel la solution aqueuse comprend un tampon de 1 à 5,5 pKa.

8. Réactif selon la revendication 7, dans lequel le tampon est au moins un tampon choisi dans le groupe constitué de : acide citrique-NaOH, acide citrique-citrate de sodium, phosphate monopotassique-phosphate disodique, phosphate monopotassique-NaOH, acide citrique-phosphate disodique, phtalate monopotassique-NaOH, acide succinique-NaOH, acide lactique-NaOH, acide ε-aminocaproïque-HCl, acide fumarique-HCl, β-alanine-NaOH et glycine-NaOH.

9. Réactif selon la revendication 1, dans lequel la concentration finale du colorant est de 0,1 à 100 ppm.

10. Réactif selon la revendication 1, dans lequel la concentration finale de tensioactif cationique est de 10 à 30 000 mg/l.

11. Réactif selon la revendication 7, dans lequel le tampon existe de 10 à 500 mM dans l'échantillon.

12. Procédé de détection et de comptage des bactéries comprenant les étapes suivantes consistant à :
(1) mélanger un échantillon d'urine avec le réactif tel que décrit à l'une quelconque des revendications 1 à 11 ;
(2) introduire l'échantillon ainsi traité dans une partie de détection d'un cytomètre de flux et irradier les cellules des bactéries colorées une à une avec une lumière afin de mesurer la lumière diffusée et la lumière fluorescente émise à partir de chacune des cellules ; et
(3) discriminer les bactéries des autres composants conformément à une intensité d'un signal de lumière diffusée et à une intensité d'un signal de lumière fluorescente ou une largeur d'impulsion réfléchissant la longueur des particules pour compter le nombre des bactéries.

13. Procédé selon la revendication 12, dans lequel l'étape (3) de discrimination et de comptage des bactéries est réalisée conformément à au moins une combinaison choisie parmi les combinaisons suivantes :
(i) une intensité de lumière diffusée vers l'avant et une largeur d'impulsion de lumière diffusée vers l'avant ;
(ii) une intensité de lumière diffusée vers l'avant et une intensité de lumière fluorescente ; et
(iii) une largeur d'impulsion de lumière diffusée vers l'avant et une intensité de lumière fluorescente.
